# EUROPEAN PATENT APPLICATION

(11) **EP 0 792 650 A2**
(43) Date of publication of application: **03.09.1997**
(21) Application number: 96118869.5
(22) Date of filing: 26.11.1996
(51) Int. Cl.: A61L 2/10

(54) **Multiple application sterilizer**

(30) Priority: 29.02.1996 AR 33559496
(71) Applicant: Altieri, Guillermo Antonio, 5515 Maipu, Pcia. de Mendoza (AR)
(72) Inventor: Altieri, Guillermo Antonio, 5515 Maipu, Pcia. de Mendoza (AR)
(74) Representative: LOUIS, PÖHLAU, LOHRENTZ & SEGETH

(57) **Abstract**

This invention refers to a portable sterilizing apparatus, particularly to an apparatus for sterilizing footwear in general with the aim to eliminate disagreeable odours. It has been attempted to solve this problem through the use of various products, which have to be spread inside shoes and which eventually penetrate the material itself.
This invention uses the proved germicidal property of ultraviolet light. For this, the apparatus of simple construction, is made up of a box within which is mounted an integrated circuit, fed by a 12 v current , and supplied by a transformer connected, on one hand, to an input of the circuit, and on the other hand, to the home power system. The circuit is made up of a power output for pairs of connecting terminals of ultraviolet light tubes or lamps, which have to be placed in the footwear in order to comply with its germicidal function. Furthermore, the circuit comprises a timer to give time for the tubes to be lit and a reset switch for lighting the latter manually by use of a button, in case the process needs to be repeated.

## Description

This invention refers to a novel sterilizing apparatus of multiple applications, and in particular, to an apparatus for sterilizing footwear through the use of ultraviolet light, whose characteristics or germicidal properties are widely known and have been proven in practice.

The main object of the apparatus of the invention is to eliminate disagreeable odours which emanate from inside footwear. It offers the possibility of being applied in places such as wardrobes, where clothing, because of its frequent use, absorbs undesirable odours of sweat. To comply with its purpose, the apparatus causes, through the exposure of footwear or of diverse clothing to the action of ultraviolet light, an energetic chemical action on the bacteria which produce these odours, and which are generated by substances secreted by the sweat glands which are generally absorbed into all clothing, and, particularly, footwear.

This well known problem has been the cause of great concern because of the discomfort it causes, and attempts have been made to solve this through the application of various products which act directly on the area which generate these odours in various articles of clothing and inside all shoes or gym shoes. These products, which are commercially known, destroy bacteria and consist basically in the deodorizing action of powders of a particular composition which necessarily has to be incorporated into clothing or spread inside footwear, a procedure which users often object to because the same have a residual effect where they are applied and which over time alters the normal appearance of the clothing or footwear, as it penetrates or impregnates the material (leather, cloth, etc.).

Another method for solving this problem is through the use of ozone generating devices, whose deodorizing effects are known and which can be applied in rooms, wardrobes or such like, but which have little effect for eliminating odours from inside footwear because for these devices to carry out their deodorizing effect it is necessary to make them work directly inside the footwear where the bacteria are to be found in places of difficult access.

In order to solve this problem effectively and at the same time offer users a simple and practical solution, the invention proposes a sterilizing apparatus based on the use of ultraviolet light which produces an active destructive effect on the bacteria and which is made up of, at least, a pair of tubes or lamps of ultraviolet irradiation to be placed respectively inside the corresponding shoes, different types of footwear, or in areas for the treatment of said odours, with the purpose of reaching hidden areas where bacteria might be found for their elimination, based on the germicidal properties of ultraviolet light.

The proposed apparatus is portable, and is basically made up of a box in which an integrated circuit is mounted and a power-supply transformer for the same, connected directly to the home power system. This circuit has power supply outlets of the transformed electrical power to 12 v. connected to pairs of terminals, secured to a wall of the box and on view to the user. This terminal allows pairs of tubes, or lamps, to be connected through the corresponding wires for the irradiation of ultraviolet light inside the footwear to be treated.

Pairs of tubes, fed by the integrated circuit, are turned on for a prefixed period of time, for example one minute, to carry out its germicidal effect, and for this purpose the circuit comprises a TIMER for keeping on the lighting the tubes and a reset switch for re-lighting the tubes, manually operable by a button mounted on the outside of the box, which allows the timer to be brought back to zero if it were necessary to repeat the sterilizing treatment of the footwear.

The apparatus can be manufactured so as to take up very little space, and the tubes of ultraviolet light, in order to effectively carry out their radiation are held up by their respective supports, which keep them separated by millimetres from the bottom of the shoes or the surface where they are placed so that the ultraviolet radiation is circumferential and reaches all the places where the bacteria might shelter.

It should be noted that the apparatus of the invention, because of its various applications, conforms a sterilizing unit which effectively completes a function which the wide range of devices, products, etc, destined to combat bacteria which produce undesirable emanations, currently lack.

Therefore, the present invention refers to a sterilizing apparatus of multiple uses, and in particular for sterilizing footwear, which is made up of a portable box within which an integrated circuit is mounted and provided with a timer and at least a pair of ultraviolet irradiation tubes, and a reset switch for said timer, controlled by a button mounted on the outside of said box, so as to be able to re-light the tubes for a new period of time, said box having a terminal for connection of the tubes fed by an outlet of said circuit whose input is connected to an electrical transformer of the home power system, said tubes being provided with the corresponding supports so as to keep the distance from the bottom of the footwear and to allow a circumferential irradiation of the ultraviolet light inside the latter.

The invention has equally on view other accessories which will be understood in the course of the present report.

For this invention to be clearly understood and put into practice with ease, it has been shown in an example and in one of its preferred embodiments, and in the drawings filed along with the present specification, in which:

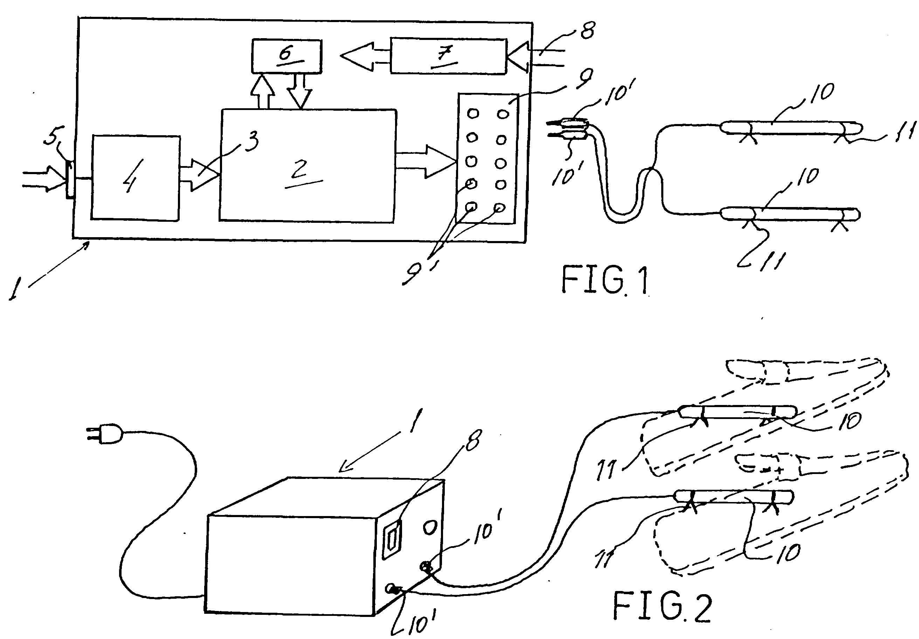

Fig. 1 is a schematic and block representation of the various components of the footwear sterilizing apparatus of the invention.
Fig. 2 is a perspective view representation of the apparatus of the invention with the ultraviolet light irradiation tubes applied to footwear.

In all the aforementioned drawings, the same reference numerals indicate equal or corresponding parts.

As shown in the drawings, the portable sterilizing apparatus of the invention comprises a portable box 1, inside which is mounted an integrated circuit 2, an input 3, and a transformer 4 connected, on one hand, by a plug 5 secured to the wall of the box 1 to the home power system, and on the other hand, to the referred input 3 of circuit 2 to supply the same with a 12 v electrical current.

The integrated circuit 2 incorporates a timer 6 and a reset switch 7, this latter being manually operated by a button 8 which can be seen in box 1, this latter having a terminal 9 with a series of pairs of terminals 9', for the connection of corresponding wires 10' of ultraviolet light tubes or lamps 10 each of which has a support 11 to keep its distance from the bottom of the shoes or from the surface to be treated. The terminals 9' are directly connected to the output of the integrated circuit 2.

The modus operandi of the apparatus of the invention is simple, as the ultraviolet light tubes 10 are supported by supports 11 on the bottom of the corresponding shoe, and once the transformer has been connected 4 to the line, the timer 6 which sets the time the tubes 10 will be lit 10 (for example one minute), which produces the sterilizing effect, and later switch off automatically. To repeat this procedure the user presses the button 8 so that the timer reset switch goes back to zero, so as to light the tubes 10 and start the procedure over again.

The invention as described is apparent and does not require any futher explanation for those skilled in the art.

This invention is clearly specified in the appended claims.

## Claims

1. A sterilizing apparatus of multiple application, particularly, to sterilize footwear wherein a portable box, within which is mounted an integrated circuit supplied with a timer which controls the amount of time the apparatus is switched on, at least a pair of ultraviolet light irradiation tubes, and a reset switch for said timer, controlled by a button mounted on the outside of said box, for resetting for a new period of time the lighting up of the tubes, said box having a terminal for the connection of the tubes supplied by an outlet of said circuit, which input is connected to an electrical transformer of the home power supply, through a wall of the box, said tubes being provided with the corresponding supports for keeping their distance from the bottom of the footwear and to allow a circumferential irradiation of the ultraviolet light inside this latter.

2. An apparatus according to claim 1, wherein said terminal is made up of a plurality of pairs of connection terminals for corresponding pairs of ultraviolet light tubes, to be able to use the same in different pairs of shoes or areas to be sterilized.
